# EUROPEAN PATENT APPLICATION

(11) **EP 4 696 260 A1**
(43) Date of publication of application: **18.02.2026**
(21) Application number: 25190988.3
(22) Date of filing: 22.07.2025
(51) Int. Cl.: A61B 18/02, A61B 18/08, A61B 90/00

(54) **CRYOABLATION APPARATUSES AND RELATED METHODS FOR NEEDLE REPOSITIONING**

(30) Priority: 12.08.2024 US 202418800766
(71) Applicant: Varian Medical Systems, Inc., Palo Alto, CA 94304 (US)
(72) Inventor: ZHANG, Hongxuan, Austin, 78750 (US)
(74) Representative: Mathisen & Macara LLP

(57) **Abstract**

A method of positioning a cryoprobe (600) includes receiving a cryoprobe repositioning selector (602) indicating a desire to reposition a cryoprobe relative to a target tissue; obtaining cryoprobe operating information from one or more sensors positioned in the cryoprobe (604), wherein the cryoprobe operating information characterizes one or more operating parameters of the cryoprobe; energizing a heater in the cryoprobe to heat a tip of the cryoprobe to a predetermined cautery temperature threshold (606); and displaying a cautery indicator when the tip of the cryoprobe is greater than or equal to the cautery temperature threshold (616).

## Description

### FIELD

The present disclosure relates to cryoablation apparatuses and related methods for the repositioning of needles during a cryoablation treatment. More particularly, the present disclosure relates to apparatuses and methods for cauterization during needle repositioning.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through a probe in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

Before or during a cryoablation treatment, it may be desirable to re-position the needle relative to the target tissue. Such re-positioning may be desirable for a variety of reasons. In some instances, it is determined that the needle is not positioned correctly or as desired relative to the target tissue. In other instances, it may be determined that the needle is located too close to or in proximity to a healthy tissue or a sensitive tissue or body structure. In still other instances, it may be determined that the iceball is not growing or achieved a size or shape as anticipated. Still further, the needle may be moved at a conclusion of a cryoablation treatment.

Existing cryoablation apparatuses and methods of use have drawbacks in instances in which a needle is moved after being placed at or near a target tissue. In instances in which the target tissue is an abnormal tissue such as a cancerous tumor, the movement of the needle may transfer abnormal tissue to a neighboring healthy tissue or to a location absent of the abnormal tissue. Such a transfer of the abnormal tissue is undesirable. In other instances, the insertion of the needle may cause bleeding and such bleeding may cause undesirable effects on the patient and/or may cause additional recovery from the treatment.

There exists a need, therefore, for improved cryoablation apparatuses and related methods of use the address these drawbacks. The cryoablation apparatuses of the present disclosure are improvements over existing apparatuses and methods by reducing a likelihood of the transfer of abnormal tissue and/or reducing bleeding that may occur after a needle is moved during a cryoablation treatment.

### SUMMARY

This section provides a general summary of the disclosure, and is not a comprehensive disclosure of its full scope or all of its features.

The present invention provides a cryoablation system or cryoablation probe as defined in the appended claims. In another aspect, the present invention provides a method for positioning a cryoprobe as defined in the appended claims. The method may include receiving a cryoprobe repositioning selector indicating a desire to reposition a cryoprobe relative to a target tissue, and obtaining cryoprobe operating information from one or more sensors positioned in the cryoprobe. The cryoprobe operating information may characterize one or more operating parameters of the cryoprobe. The method may also include energizing a heater in the cryoprobe to heat a tip of the cryoprobe to a predetermined cautery temperature threshold, and displaying a cautery indicator when the tip of the cryoprobe is greater than or equal to the cautery temperature threshold.

In one aspect, the method may include cooling the tip of the cryoprobe to a temperature less than or equal to a movement temperature, and displaying a movement indicator when the tip of the cryoprobe is less than or equal to the movement temperature. The target movement temperature may be 51 degrees Celsius or lower. For example, the target movement temperature may be from 1-51 degrees Celsius, such as from 30-50 degrees Celsius, for example, from 36-50 degrees Celsius.

In another aspect, the cautery temperature threshold may be 70 degrees Celsius or greater. In some embodiments the cautery temperature threshold may be greater than 80 or 90 or 100 degrees Celsius. Suitably, the cautery temperature threshold is no greater than 200, or 175, or 150 or 125 degrees Celsius. The cautery temperature threshold maybe from 70 to 100 degrees Celsius, or 80-100 degrees Celsius or 90 to 100 degrees Celsius.

In another aspect, the tip of the cryoprobe may be maintained at the cautery temperature threshold for a period of at least 5 seconds. The tip of the cryoprobe may be maintained at the cautery temperature threshold for a period of greater than 20 seconds. The tip of the cryoprobe may be maintained at the cautery temperature threshold for a period of from 5 seconds to 20 seconds.

In another aspect, the one or more sensors may include at least one impedance sensor and/or at least one temperature sensor, and the cryoprobe operating information comprises impedance data and/or temperature data.

In another aspect, the method may also include determining a completion of cauterization of the target tissue at the tip of the cryoprobe based on the impedance data and the temperature data.

In another aspect, the method may include displaying a movement indicator indicating when cautery of the target tissue is complete and the cryoprobe can be moved to a new location relative to the target tissue.

In another aspect, the step of energizing the heater in the cryoprobe may include adjusting a power delivered to the heater based on the impedance data and the temperature data.

In another aspect, the one or more sensors may include a plurality of impedance sensors and a plurality of temperature sensors. The plurality of impedance sensors may be spaced apart from each other to provide impedance data for a plurality of locations along a length of the tip of the cryoprobe and the plurality of temperature sensors may be spaced apart from each other to provide temperature data for a plurality of locations along the length of the tip of the cryoprobe.

In another aspect, the method may include determining a cauterization length based on the impedance data and the temperature data, the cauterization length characterizing a length of the tip of the cryoprobe that reaches the cautery temperature threshold for a cautery period of time.

In some embodiments a cryoablation system is provided. The cryoablation system may include a cryoprobe assembly with a needle, a cryogen delivery apparatus fluidly connected to the cryoprobe assembly, and a cryoablation control apparatus comprising a power supply, a user input/output unit, at least one processor, and memory, wherein executable instructions stored in the memory cause the at least one processor to: receive a cryoprobe repositioning selector indicating a desire to reposition the cryoprobe, obtain cryoprobe operating information from one or more sensors positioned in the needle, energize a heater in the needle to heat the needle to a predetermined cautery temperature threshold, and display a cautery indicator when a temperature of the needle is greater than or equal to the cautery temperature threshold. The cryoprobe operating information characterizing one or more operating parameters of the needle. The needle may be configured as a pointed tool having an outer diameter in a range of about 1 mm to about 4 mm. The heater may be positioned at or near the tip of the needle. The heater may be coupled to the cryoablation control apparatus and the power source provided therein. The heater may be any suitable heater, for example, the heater may be a resistive heater. The cryoablation system may further comprise a cryoprobe monitor. The cryoprobe monitor may be electrically coupled to the cryoablation control apparatus. The cryoprobe monitor may provide the cryoprobe operating information to the cryoablation control apparatus. The heater may be coupled to the cryoprobe monitor.

In one aspect, the executable instructions may further cause the processor to: cool the tip of the cryoprobe to a temperature less than or equal to a movement temperature; and display a movement indicator when the tip of the cryoprobe is less than or equal to the movement temperature. Upon the initiation of a freezing cycle, the cryoablation control apparatus may cause the cryogen to be moved through a cryogen flow path, that includes a cryogen supply line from a cryogen source, through the cryogen supply line to the cryoprobe 112. The cryogen may then flow back to the cryogen source via a cryogen return line or may be exhausted from the cryoablation system using a suitable exhaust valve. The cryogen supply line may be a flexible tube or other conduit that may include multiple lumens to allow the cryogen to flow in a supply direction to the cryoprobe and separately in a return direction away from the cryoprobe.

In another aspect, the cautery temperature threshold may be at least 70 degrees Celsius or greater. The cautery temperature threshold greater than 80 or 90 or 100 degrees Celsius. Suitably, the cautery temperature threshold is no greater than 200, or 175, or 150 or 125 degrees Celsius. The cautery temperature threshold may be 70-100 degrees Celsius, such as 80-100 degrees Celsius or 90-100 degrees Celsius.

In another aspect, the temperature of the needle may be maintained at the cautery temperature threshold for a period of at least 5 seconds. The temperature of the needle may be maintained at the cautery temperature threshold for a period of at least 20 seconds. Suitably, the temperature of the needle is maintained at the cautery temperature threshold for a period of between 5 and 20 seconds.

In another aspect, the one or more sensors may include at least one impedance sensor and/or at least one temperature sensor, and the cryoprobe operating information may include impedance data and temperature data.

In another aspect, the executable instructions may further cause the processor to determine a completion of cauterization based on the impedance data and the temperature data.

In another aspect, the executable instructions may further cause the processor to display a movement indicator indicating when cautery of the target tissue is complete and the needle can be moved to a new location.

In another aspect, the step of energizing the heater in the needle may include adjusting a power delivered to the heater based on the impedance data and the temperature data.

In another aspect, the one or more sensors may include a plurality of impedance sensors and/or a plurality of temperature sensors, the plurality of impedance sensors spaced apart from each other to provide impedance data for a plurality of locations along a length of the needle, the plurality of temperature sensors spaced apart from each other to provide temperature data for a plurality of locations along a length of the needle. The cryoprobe may comprise 2, 3, 4 or more impedance sensors. The cryoprobe may comprise 2, 3, 4 or more temperature sensors.

In another aspect, the executable instructions may further cause the processor to determine a cauterization length based on the impedance data and the temperature data, the cauterization length characterizing a length of the needle that reaches the cautery temperature threshold for a cautery period of time.

The cryoablation system may further include a user interface, which user interface may be used to disclose and/or to control the functionality of the cryoablation apparatus. The user interface may display a current operating function of the cryoprobe, for example the user interface may indicate when the cryoprobe is performing a freezing cycle, and/or when the cryoprobe is performing an active heating cycle. The user interface may indicate when the cryoprobe has already performed a cauterization cycle and is ready to be moved.

In another aspect the present invention provides a cryoprobe, including a needle and a heater positioned at or near the tip of the needle, the needle including one or more impedance sensor and/or one of more temperature sensor. The heater may be any suitable axial length according to the desired application. For example, the heater may extend longitudinally in an axial direction along the length of the needle. In some examples the heater is from 20-30 mm long. The needle of the cryoprobe may be configured as a pointed tool suitably having an outer diameter in a range of 1 mm to 4 mm. The cryoprobe may include a cryogen flow path, that includes a cryogen supply line, extending into the cryoprobe and a cryogen return line or a cryogen exhaust valve. The cryogen supply line may be a flexible tube or other conduit that may include multiple lumens to allow the cryogen to flow in a supply direction to the cryoprobe and separately in a return direction away from the cryoprobe. Suitably, the needle of the cryoablation probe comprises a shell and a tip. The cryoprobe may comprise 2, 3, 4 or more impedance sensors. The cryoprobe may comprise 2, 3, 4 or more temperature sensors. The one or more impedance sensor and/or temperature sensor may be positioned at one or more positions along the length of the shell. Suitably, the cryoablation probe comprises a plurality of impedance sensors spaced apart along the length of the shell. Suitably the cryoablation prove comprises a plurality of temperature sensors spaced apart along the length of the shell. The plurality of impedance and/or temperature sensors may be positioned in an array of different axial or radial positions along the shell.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an example cryoablation apparatus in accordance with some embodiments of the present disclosure.
FIG. 2 is an illustration of another example cryoablation apparatus in accordance with some embodiments of the present disclosure.
FIG. 3 is an illustration of an example graphical user interface is included in some embodiments of the present disclosure.
FIG. 4 is a sectional side view of an example cryoprobe needle that is used on some cryoablation apparatuses of the present disclosure.
FIG. 5 is a diagram showing an example procedure and a corresponding impedance, temperature, and power signal profile that may be obtained and/or delivered by aspects of a cryoablation apparatus during an example re-positioning of an ablation needle in accordance with some embodiments of the present disclosure.
FIG. 6 is an example method of performing a cautery procedure in accordance with some embodiments of the present disclosure.
FIG. 7 is a block diagram illustrating an example computing device that may be used to perform the methods of the present disclosure.
FIG. 8 is a graph illustrating aspects of performing a cautery procedure in accordance with some embodiments of the present disclosure.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In some embodiments of the present disclosure, a cryoablation apparatus is provided that may be used to perform cryoablation treatments. The cryoablation apparatus may include one or more features or may be operated according to one or more methods to re-position the needle of the cryoprobe while minimizing a likelihood of transferring abnormal tissue cells (e.g., cancer cells) to adjacent tissues or adjacent portions of the body. The cryoablation apparatuses and methods of the present disclosure may utilize a cautery feature and functionality to raise a temperature of the needle at the target tissue to cauterize the tissue surrounding the needle and then lower the temperature of the needle before indicating to the user that movement of the needle can be performed.

It may be desirable to move a needle during a cryoablation treatment because the current position of the needle may not be located in a position relative to the target tissue such that the iceball that is produced during a freezing cycle would destroy the target tissue. In other circumstances, the needle may be positioned too close to healthy tissues or other body structures. In still other circumstances, the iceball may not form as predicted during a freezing cycle and the needle needs to be moved to perform an additional or supplemental freezing cycle in a new position. Still further, the freezing cycle may have been completed and it is time to withdraw the needle from the inserted position. In each of these circumstances or others, it may be desirable to move the needle from a first position to a second position.

In the various embodiments described below, the cryoablation apparatus may minimize a likelihood that abnormal cells are moved from the first position to neighboring tissues or body structures by cauterizing the tissue surrounding the needle before the needle is moved. Such cauterization may be achieved by raising the temperature of the needle to a temperature of 80 degrees Celsius or greater and holding this cauterization temperature for a period of about 5 to about 20 seconds. After such cauterization is performed, the cryoablation apparatus may indicate to the user that the needle may be moved from the initial position to a second position.

The cryoablation apparatuses of the present disclosure are improvements over existing cryoablation apparatuses and methods. Existing cryoablation apparatuses and methods may not provide suitable elevated temperatures to perform cauterization. In addition, existing apparatuses and methods rely on the experience and skill of the user such that real-time control and feedback is not provided to the user. The cryoablation apparatuses of the present disclosure provide feedback to the user during cautery procedures to positively indicate that a suitable cautery procedure has been performed and/or that the needle is ready to be moved to a new location. Furthermore, the apparatuses and methods of the present disclosure can obtain real-time measurements to accurately and efficiently control the operation of the cryoablation apparatuses to achieve cautery temperatures and movement temperatures as will be further described below.

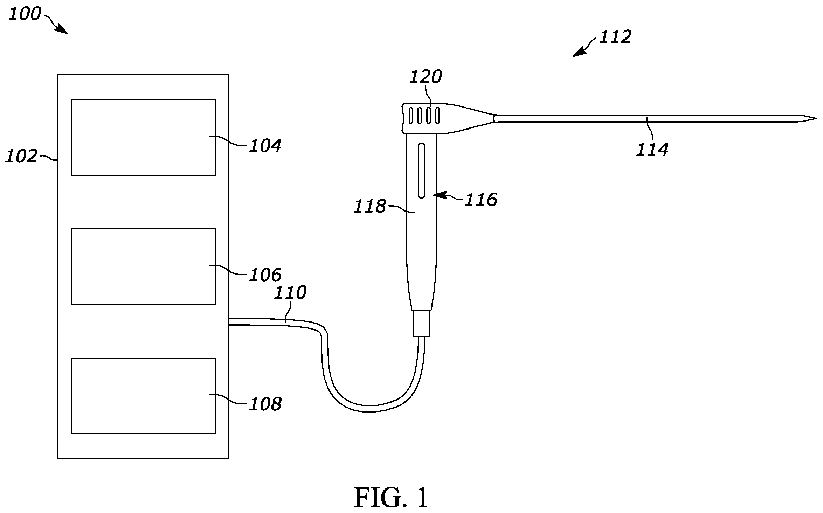

Referring now to FIG. 1, an example cryoablation apparatus 100 is shown. The cryoablation apparatus 100 may include a cryoablation console 102, and a cryoprobe 112. The cryoablation console 102 may include a cryo-controller 104, a cryogen delivery apparatus 106 and a cryogen source 108. The cryo-controller 104 may include a computing device or other controller that can be used to control delivery of a cryogen from the cryogen source 108 to the cryoprobe 112 using the cryogen delivery apparatus 106. The cryogen source 108 may be a suitable Dewar or other container that can be filled with a cryogen. The cryogen delivery apparatus 106 may include a pump, one or more valves, and other suitable fluid delivery devices to fluidly connect the cryogen source to the cryogen line 110 of the cryoprobe 112. Upon the initiation of a freezing cycle, the cryo-controller 104 may cause the cryogen to be moved through a cryogen flow path that includes a cryogen supply line from the cryogen source through the cryogen line 110 to the cryoprobe 112. The cryogen may then flow back to the cryogen source via a cryogen return line or may be exhausted from using a suitable exhaust valve.

The cryogen line 110 may be a flexible tube or other conduit that may include multiple lumens to allow the cryogen to flow in a supply direction to the cryoprobe 112 and separately in a return direction away from the cryoprobe 112. The cryogen line 110 may of sufficient length to allow the console 102 to be positioned near the patient in a treatment room and to allow the patient to be moved into and out of an imaging device. In some examples, the cryogen line may be at least about 3.65 meters (12 feet) in length. In other examples, the cryogen line 110 may have other lengths.

The cryogen line 110 fluidly couples the cryogen source 108 to the cryoprobe 112. The cryoprobe 112 may include a needle 114. The needle 114 may be a pointed cylindrical tool or other elongated member that is configured to be inserted into patient tissue and be positioned at or near the target tissue during treatment. The needle 114 may be configured as a pointed tool having an outer diameter in a range of about 1 mm to about 4 mm. The cryoprobe 112 may also include a handle 116. The handle 116 may be configured with a first (or proximate) portion 118 and a second (or distal) portion 120. The first portion 118 may be substantially aligned with the cryogen line 110 and the second portion 120 may offset at an angle relative to the first portion 118. The offset angle between the first portion 118 and the second portion 119 may be about 90 degrees to define a right angle handle. In other example, the first portion 118 and the second portion 120 may be offset at different angles.

In some examples, the handle 116 may include a vacuum chamber positioned at or near an outside surface of the handle 116. The vacuum chamber may insulate the exterior of the handle from the extremely low operating temperatures of the cryogen that moves through the handle to the cryoprobe 112. This may allow an operator to touch or otherwise manipulate the cryoprobe 112 during treatment.

The needle of the cryoprobe 112 may also include a heater positioned at or near the tip of the needle. The heater may be used to raise a temperature of the needle. The heater may be coupled to the console and suitable power source provided therein. The cryo-controller 104 may control a power signal delivered to the heater to raise a temperature of the needle. The temperature may be raised to a suitable cauterization temperature in a range of about 70 degrees Celsius to about 100 degrees Celsius. In other example, the cauterization temperature may be about 80 degrees Celsius or greater. In yet other examples, the cauterization temperature may be about 90 degrees Celsius to about 100 degrees Celsius.

While not shown, more than one cryoprobe 112 may be coupled to the console 102. Multiple cryoprobes 112 may be used during a single cryoablation treatment in combination. The console 102 may be configured to deliver cryogen to each of the multiple cryoprobes 112. The cryoprobes 112 may be similar to each other or may be different to produce iceballs of different sizes and shapes so as to freeze and destroy the target tissue.

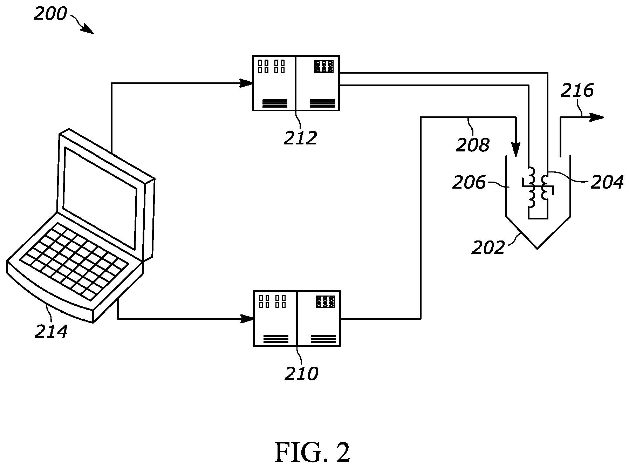

Referring now to FIG. 2, another example cryoablation apparatus 200 is shown. In this example, the cryoablation apparatus 200 may include a cryoprobe 202 operably coupled to a cryoablation control apparatus 214. The cryoablation control apparatus 214 may include a computing device such as a laptop, tablet, controller, programmable logic controller, smart device, or the like. The cryoablation control apparatus 214 may include a processor and memory. The memory may include executable instructions stored on the memory that may cause the cryoablation apparatus 200 to perform the functions and/or methods described herein.

The cryoablation apparatus 200 may include a cryogen delivery apparatus 210 and a cryoprobe monitor 212. The cryogen delivery apparatus 210 may include valves, pumps, and/or other cryogen delivery devices that may deliver a cryogen (e.g., liquid nitrogen) to the cryoprobe 202. The cryogen delivery apparatus 210 may be electrically coupled and/or controlled by the computing device of the cryoablation control apparatus 214. The cryogen delivery apparatus 210 may cause cryogen to flow to the probe in a supply line 208 and the be removed and/or vented from the cryoprobe 202 via a return or exhaust line 216.

The cryoprobe monitor 212 may also be electrically coupled to the computing device of the cryoablation control apparatus 214. The cryoprobe monitor may be an electrical bus, a data acquisition unit, a computing device, a control board, circuit or other device that may receive cryoprobe operating information from the cryoprobe 202. The cryoprobe operating information may be, for example, temperature and/or impedance information that may be obtained using a sensor, thermocouple, temperature sensor, impedance sensor, or the like. The cryoprobe operating information may correspondence to a temperature and/or impedance obtained at one or more locations on or in the needle of the cryoprobe 202. The cryoprobe monitor 212 may provide the cryoprobe operating information to the cryoablation control apparatus 214.

The cryoprobe 202 may be similar to the cryoprobe assembly 112 previously described. In this example, the cryoprobe 202 may include a heater 204 positioned in a needle 206 of the cryoprobe 202. The heater 204 may be a resistive heater in some examples. The heater 204 may be coupled to the cryoprobe monitor 212. A temperature of the heater 204 and/or of the needle 206 may be obtained by the cryoprobe monitor 212. The cryoprobe monitor 212 may also provide a power signal to the heater 204. The cryoprobe monitor 212 may include a power supply or be coupled to a power supply to provide a desired power signal to the heater 204. The cryoprobe monitor 212 may adjust, change, and/or modulate the power signal to the heater 204 based on the cryoprobe operating information previously described. The cryoprobe monitor 212 and/or the cryoablation control apparatus 214 may adjust, change or modulate a suitable power signal to the heater 204 to cause the needle 206 of the cryoprobe 202 to achieve a desired cauterization temperature for a desired period of time.

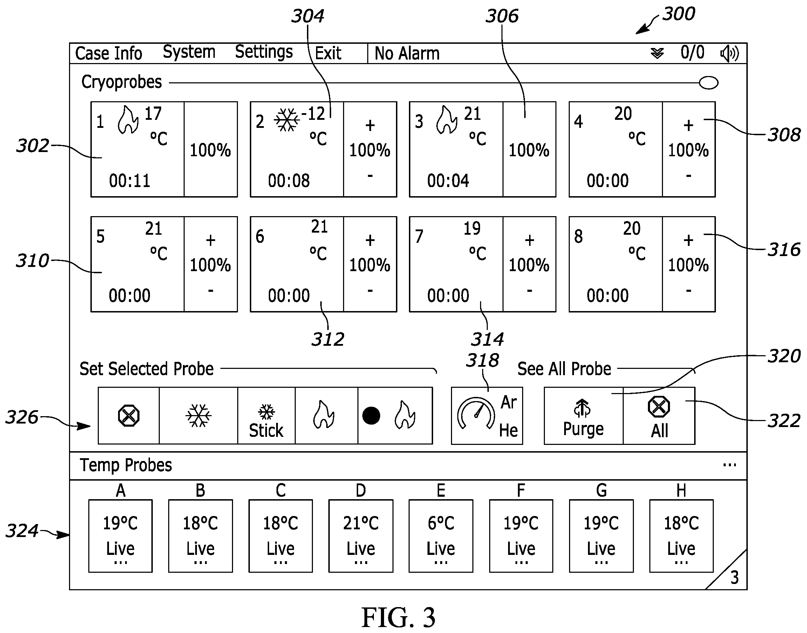

In some examples, the cryoablation control apparatus 214 may include and/or be coupled to a display. The display may be a computer display, LCD screen, LED screen, or other display device. The cryoablation control apparatus 214 may cause a user interface to be displayed during use of the cryoablation apparatus 100, 200. In one example as shown in FIG. 3, a user interface 300 may include one or more cryoprobe panels 302, one or more probe function buttons 316, a cryogen selector 318, one or more all-probe selectors, and one or more temperature panels 324.

The user interface 300 may be used to display and to control the functionality of the cryoablation apparatus 100, 200. The cryoprobe panels 302 may display a current operating function the cryoprobe 202 and other information regarding the operation of the cryoprobe 202. For example, the cryoprobe panel 302 may include a background color that may indicate a function of the cryoprobe. The cryoprobe panel 302 may include blue background to indicate that the cryoprobe 202 is performing a freezing cycle. In addition to the background color, an icon may be displayed in the cryoprobe panel 302. The icon may be snowflake as shown in the second cryoprobe panel 304. The background may be displayed as red and include a flame icon when the cryoprobe is performing an active heating function of the cryoprobe 202 as shown in example cryoprobe panel 306. The background may be displayed as green when the cryoprobe 202 has already performed a cauterization cycle and is ready to be moved as shown in cryoprobe panel 302.

Each of the cryoprobe panels 302 to 316 may include such display functionality. In addition to the icons and background colors as described above, the cryoprobe panel 302 may display a timer that displays a length of time of the current cycle (e.g., freezing, heating, cauterization, etc.). The cryoprobe panel 302 may also display a current temperature of the needle of the cryoprobe 202. In this example, the user interface 300 includes eight cryoprobe panels 302, 304, 306, 308, 310, 312, 314, and 316. In other examples, the user interface 300 may include more or less than eight cryoprobe panels. In addition to other background colors, other icons or indicators may also be displayed.

The probe function selectors 326 may be displayed in the user interface 300 and may allow a user to perform a desired function of the cryoprobe 202. The selectors 326 may include a stop selector, a freezing selector, a needle stick selector, a thaw selector, and a cauterization selector. The stop selector may be selected by the user and stop the current active function of the cryoprobe 202. The freezing selector may be selected by the user and may cause a freezing cycle to begin. Such selection may initiate a flow of cryogen to the needle of the cryoprobe 202, for example. The needle stick selector may initiate a freezing cycle that may be performed to cause the ablation probe needle to stick to the surrounding tissue. Such a process may be performed at the start of an ablation treatment to limit and/or prevent movement of the needle during a treatment.

The thaw selector may initiate a thaw cycle whereby a heater may be energized to heat the needle of the probe after a freezing cycle is performed. The cauterization selector may initiate a cauterization cycle to be performed that may cauterize the tissue surrounding the needle.

As further shown in FIG. 3, the interface 300 may include a cryogen or fluid selector 318. [Inventors: please indicate the functionality of this button in the interface]. The interface 300 may also include purge selector 320 and all-off selector 322. The purge selector 320 may initiate a purge cycle to be performed. During such a cycle, a fluid may be passed through the cryogen flow path in the supply cable and/or the cryoablation probe to clear cryogen, ice, or other materials that may be deposited or may have formed in the cryogen flow path during use. The all-off selector 322 may cause each of the ablation probes that are coupled to the cryoablation apparatus to be turned off. Any cycle that may be performed or in-process is stopped when such selector may be selected by a user.

The user interface 300 may include other buttons or input selectors in addition to the ones shown or described above. As will be further described below, the user interface 300 may indicate to a user when a cautery cycle has started and/or is complete. The user interface 300 may indicate to a user when a cautery cycle has started using an icon, color, and/or other indicators on the corresponding cryoprobe panel 302. The user interface 300 may also indicate to a user when the cautery cycle is complete and whether the user can move the needle of the cryoprobe to a new location using an icon, color, and/or other indicators on the corresponding cryoprobe panel 302. Such a movement indicator may be displayed when the needle has achieved a predetermined cautery temperature and has maintained the predetermined cautery temperature for a predetermined cautery period.

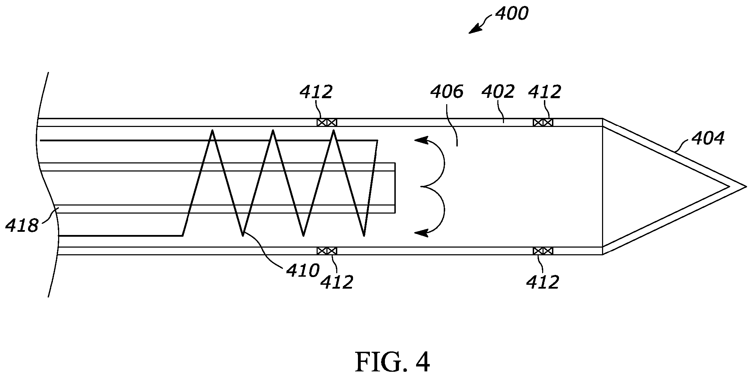

Referring now to FIG. 4, an example cryoprobe needle 400 is shown. In this example, the needle 400 may include a shell 402 and tip 404. The shell 402 may be made of a suitable material such as a metal or alloy. In some examples. The shell 402 may be made of a stainless steel. In other examples, other materials may be used. The shell 402 may be a thin-walled tube that defines an inner cavity 406. The tip 404 may be tapered and positioned at a distal end of the shell 402. The tip 404 may assist in piercing and/or allowing the needle 400 to be moved into a desired position in or near the target tissue.

A cryogen supply 418 may be positioned in the inner cavity 406. The cryogen supply 418 may be axially aligned with an axis of the shell 402. The cryogen supply 418 may be configured as a cylindrical lumen configured to convey the cryogen from a cryogen source to the distal end of the needle 400. The cryogen may exit the cryogen supply 418 at or near the tip 404 of the needle 400 and then flow in a direction away from the tip 404 through a return path defined between the inner surface of the shell 402 and an outer surface of the cryogen supply 418. The flow of the cryogen may cause the temperature of the needle to drop and may move heat away from the target tissue and the distal end of the needle 400 and cause an iceball to be produced at the target tissue. The iceball may be of a sufficiently low temperature to destroy the target tissue.

A heater 410 may also be positioned in the inner cavity 406 and/or in or on the shell 402. The heater 410 may be a resistive heater that increases in temperature when a power signal is supplied to the heater 410. In other examples, other types of heaters may be used. The heater 410 may cause a temperature of the needle 400 to increase when the heater 410 is energized. The heater may cause a temperature of the heater to increase to a temperature to allow the cauterization of tissue that is at or near the distal end of the needle or at an axial location at or near the heater 410. In some example, the heater 410 may allow the needle 400 to be heated to a temperature greater than 80 degrees Celsius. In other example, the heater 410 may allow the needle 400 to be heated to a temperature greater than 90 degrees Celsius. In still other examples, the heater 410 may allow the needle 400 to be heated to a temperature greater than 100 degrees Celsius. The heater 410 may be configured to heat the needle 400 to the cautery temperature of a predetermined period of time. The period of time may correspond to a time in which the cauterization of tissues at the needle 400 occurs. In some examples, the cautery period is at least 5 seconds. In other examples, the cautery period is in a range of about 5 to 20 seconds. In still other examples, the cautery period is at least about 20 seconds.

The heater 410 may have suitable axial lengths so that the target tissue that is in contact with the needle 400 may be cauterized during a cautery cycle. As can be appreciated such a length may vary according to the size of the target tissue (e.g., tumor). In some examples, the heater 410 may have a length of about 20 mm to about 30 mm. Such a length may create or allow the needle 400 to have a cauterization length of about 20 mm to about 30 mm long. In circumstances in which the target tissue is larger than the cauterization length, the needle 400 may have to be moved in two or more steps. For example, the needle may need to cauterize the target tissue in a first position after which the needle is moved or extracted a distance corresponding to the cauterization length of the needle 400. The needle 400 may then perform another cauterization cycle at the extracted position to further cauterize the target tissue that was not cauterized when the needle 400 was in the first or initial position. In such a manner, the needle 400 can be moved from an initial position while preventing or reducing a likelihood that cells from the target tissue are moved to transported from the initial position of the needle 400 to a second position.

As further shown in FIG. 4, the needle 400 may include one or more impedance sensors 412. The impedance sensors 412 may be a suitable impedance sensor comprising one or more electrodes across which an impedance can be measured in response to an applied signal. The impedance sensors 412 may be coupled to a suitable computing device, processor, or circuit to determine an impedance at the impedance sensor 412. The impedance sensors 412 may be positioned at one or more positioned along the shell 402. In this configuration, an impedance may be measured at various locations along the needle 400. The impedance may be used to determine one or more properties of the tissue that may be in contact with the needle 400 or may surround the needle 400. For example, a progress of a cauterization cycle may be determined using the impedance sensors 412 because an impedance may change as the tissue is heated due to change in water content in the tissue. In addition, a type of tissue may be determined using the impedance measurements form the impedance sensors 412. Different tissue types may require different cauterization temperature and/or different cauterization time periods. The impedance sensors 412 may provide information to allow the cryoablation apparatuses of the present disclosure to determine and/or indicate to a user when a cauterization cycle is complete and the needle 400 may be moved.

In the example shown, the needle 400 includes four impedance sensors 412 positioned at different locations on the shell 402. In other examples, the needle 400 may include more than four impedance sensors (or less than four impedance sensors). In various examples, the impedance sensors 412 may be positioned in an array or different axial or radial positions along the shell 402 to provide information regarding a tissue, water-content, cauterization progress, or other information.

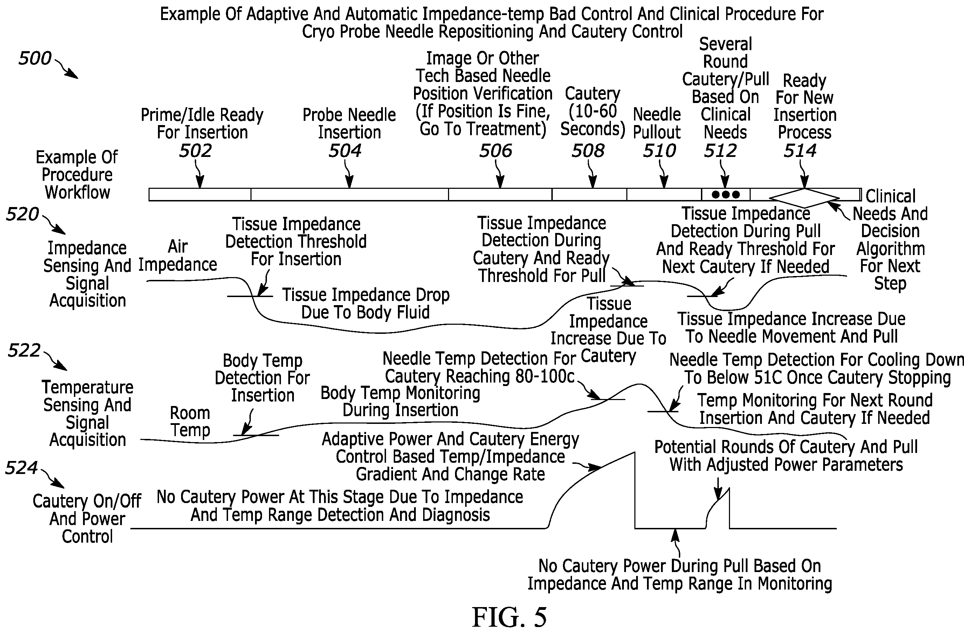

Referring now to FIG. 5, an example cryoablation procedure is illustrated that may be performed using the cryoablation apparatuses or cryoablation probes of the present disclosure. The procedure 500 illustrates one example cryoablation procedure that includes various steps 502 to 514 of an example procedure or workflow. An impedance 520 is illustrated along with example temperature 522 and an example cautery power signal 524. The corresponding lines in each of the foregoing illustrates how each may change during each of the steps of the workflow.

The procedure 500 may begin at step 502 at which time the cryoablation probe is idle and/or may be primed for insertion into a patient. At step 502, an impedance 520 is measured which corresponds to an impedance of ambient air since the probe has yet to be inserted at the target tissue. The temperature is also measured and such temperature corresponds to room temperature. The cautery power signal 524 is off and no power is being delivered to the cryoablation probe since no cauterization is being performed.

In the example procedure 500, step 504 may correspond to a period when the needle of the cryoablation probe is inserted into a patient and is guided toward the target tissue. During this period, the impedance 520 may decrease as the needle is inserted into tissue. The temperature 522 may increase due to the increase in temperature as the needle is surrounding by body tissue of the patient. The power signal 524 remains flat since no cauterization is being performed.

At step 506, an imaging process may be performed. The imaging process may correspond to obtaining a CT, MRI, ultrasound, or other image of the patient at or near the target tissue. The image may be used to confirm and/or determine a location of the needle of the cryoablation probe relative to the target tissue. If the needle is placed in a desired position, the procedure may proceed to a freezing cycle (not shown in FIG. 5). In this example, the procedure 500 is shown in which the needle is not positioned at a desired location and needs to be moved. In such an instance, a cauterization cycle is performed prior to movement of the needle to prevent or reduce a transfer of abnormal cells (i.e., cells from a tumor such as cancerous cells).

At step 508, a cauterization cycle is performed. During such a cycle, the power signal 524 may be delivered to the heater in the needle of the cryoablation probe. The heater may cause the temperature 522 at the needle to increase. The temperature may be increased to a desired or predetermined cauterization temperature range such as in a range of about 80 degrees Celsius to about 100 degrees Celsius. During this heating, the impedance 520 may also increase at the tissue. The heater in the needle may continue to heat until the cauterization temperature is reached. Once a threshold or range is achieved, the heater may maintain the needle at the cauterization temperature for a predetermined period of time. During the cauterization cycle, the impedance 520 that is measured may be used to adjust or change the power signal 524 that is delivered to the heater.

At step 510, the needle may be pulled out of the target tissue. Before the needle is moved, the cryoablation apparatus may indicate to the user that the needle may be moved. Such indicator may be displayed using the user interface 300 previously described. If the target tissue is not large such that the cauterization length or cauterization zone is able to cauterize the target tissue, the needle may be extracted from the target tissue. If, however, the cauterization zone is not large enough to cauterize the target tissue along the needle, it may be necessary to perform another cauterization cycle once the needle is extracted a distance amounting to the same as the cauterization length or less. The steps 508 and 510 may be re-performed at step 512 as may be necessary to limit or reduce a likelihood that abnormal cells are transferred to surrounding areas in the patient.

At step 514, the cryoablation probe may be ready for re-insertion. At such time, the impedance 520, the temperature 522 and the power signal 524 may be similar to the values and conditions as the original insertion at step 502. After step 514, the process 500 may be re-performed as needed until such time as it is confirmed that the needle of the cryoablation probe is positioned in a desired location relative to the target tissue.

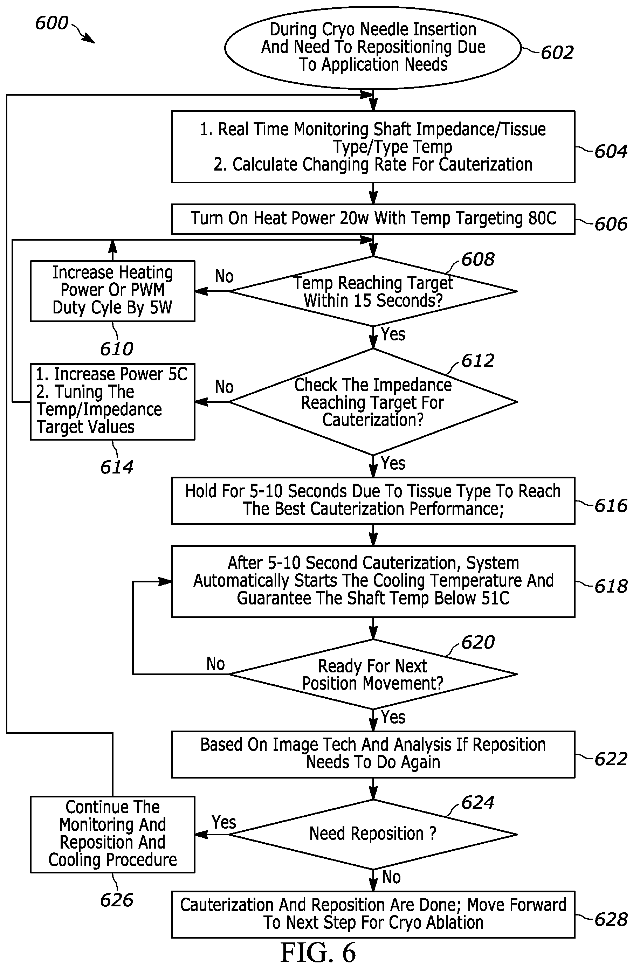

Referring now to FIG. 6, an example method 600 of repositioning a needle of a cryoprobe is illustrated. The method 600 may be performed using the cryoablation apparatus 100 or 200. For illustration purposes, the method 600 is described with reference to cryoablation apparatus 100. It should be appreciated, however, that other apparatuses or systems may be used to perform the method 600 or variations thereof.

The method 600 may begin at step 602. At step 602, it is determined that a cryoablation probe needle needs to be repositioned. At step 602, a user may indicate that a repositioning is desired by selecting a needle repositioning selector or cauterization selector on a user interface. Such user interface may be displayed and/or coupled to the cryo-controller 104. The cryo-controller 104 may receive a cryoprobe repositioning selection from the user interface. In other examples, the cryoprobe repositioning selection may be obtained from a different user interface, a button, or other control.

At step 604, the cryo-controller 104 may obtain cryoprobe operating information from one or more sensors positioned in or on the cryoprobe. Such sensors may include the sensors described with respect to needle 400. The cryoprobe operating information may characterize one or more operating parameters of the cryoprobe. The cryoprobe operating information may include, for example, a temperature or impedance at the needle of the cryoprobe. The cryo-controller 104 may use such information to determine a type of tissue at the needle and/or a power signal or power profile to use to energize the heater during a cauterization cycle.

At step 606, the cryo-controller 104 may energize the heater of the cryoprobe. The cryo-controller 104 may use the power signal or power profile determined using the cryoprobe operating information obtained at step 604. In one example, the heater may be energized using a 20 Watt power signal with a target temperature threshold of 80 degrees Celsius. In other examples, the cryo-controller 104 may deliver other power signals and/or target other cauterization temperature thresholds or cauterization temperature ranges as previously described.

At step 608, the cryo-controller 104 may determine if the heater is heating at a desired rate. It may be desirable to heat the needle of the cryoprobe within a certain period of time. In one example, it may be desired to reach the target cauterization temperature within 15 seconds. In other examples, other target periods of time may be used. If the cryo-controller 104 determines that the target cauterization temperature is reached with the desired period of time, the method may proceed to step 612. If the cryo-controller 104 determines that the target cauterization temperature is not reached within the desired period of time, the method may move to step 610.

At step 610, the cryo-controller 104 may adjust or change the power signal delivered to the heater in the needle of the cryoprobe. The cryo-controller 104 may adjust or change the power signal to increase the rate at which the needle is heating. In one example, the cryo-controller 104 may increase the power signal or increase a pulse width modulation or duty cycle delivered to the heater. In some examples, the power may be increased in predetermined increments. Such an increment may be about 5 Watt increments. In other examples, other increments may be used. The method may then return to step 608 to determine if the target cauterization temperature is reached.

At step 612, the cryo-controller 104 has determined that the target cauterization temperature has been reached. The cryo-controller 104 may also determine whether the measured temperature corresponds a target cauterization impedance. The target cauterization temperature may be correlated to or be based on a measured impedance of the tissue at the needle. Because of this relationship, it may be necessary to adjust the temperature at the needle based on a measured impedance. If the cryo-controller 104 determines that the impedance at the needle is within a desired impedance range as related to the measured temperature, the method may proceed to step 616. If the cryo-controller determines that a different temperature may be needed based on the measured impedance, the method 600 may move to step 614.

At step 614, the cryo-controller 104 may adjust or change the power signal delivered to the heater. The cryo-controller 104 may tune the power signal to achieve a desired temperature based on the measured impedance. This process may be iterative. The method 600 may perform the steps 608 through 614 based on the temperatures and impedances at the needle obtained by the cryo-controller 104. When the desired target cauterization temperature is achieved based on a measured impedance, the method 600 moves to step 616.

At step 616, the cryo-controller 104 may maintain the target cauterization temperature for a predetermined cauterization period of time. In some examples, the predetermined cauterization period of time is in a range of about 5 seconds to about 10 seconds. In other examples, the predetermined cauterization period of time is about 5 seconds to about 20 seconds. In other examples, other periods may be used.

At step 618, the cryo-controller 104 may cause cooling of the needle to begin. The cryo-controller 104 may stop power from being delivered to the heater. The cryo-controller 104 may also start cryogen to be delivered via the cryogen supply to the needle from the cryogen source 108. The cryo-controller 104 may monitor the temperature at the needle to ensure that the needle has achieved a target movement temperature. The target movement temperature may be a temperature at which the needle may be safely repositioned to a new location. In one example, the target movement temperature is a temperature of about 51 degrees Celsius or lower. The cryo-controller 104 may continue the cooling cycle until the target movement temperature is achieved.

During steps 604 to 620, the cryo-controller 104 or other computing device may be displaying a user interface, such as user interface 300, to the user. The user interface may display and/or indicate to the user that a cauterization cycle is being performed. The cauterization cycle indicator may indicate that a cauterization cycle is being completed and that the user should not move the needle until the cauterization cycle is complete. In the example user interface 300, the cryoprobe panel 302 may display an icon and may have a particular color (e.g., red) to indicate that the cauterization cycle is in progress.

At step 620, the cryo-controller 104 may determine whether it is acceptable to move the needle of the cryoprobe. The cryo-controller 104 may compare the current needle temperature to the movement temperature threshold to make this determination. If the cryo-controller 104 determines that the needle temperature is at or below the movement temperature threshold, the method may move to step 622. At such time, the cryo-controller 104 may also cause a movement indicator to be displayed to the user indicating that the needle may be safely moved. In one example, the user interface 300 may display a particular color (e.g., green) in the cryoprobe panel 302. If the cryo-controller 104 determines that the needle is not ready to be moved, the method 600 may return to step 618 and continue to monitor the temperature of the needle until it is ready to be moved.

At step 622, it may be determined whether the needle needs to be moved again after it has been repositioned. A position of the needle may be determined using imaging or other diagnostic procedures. If the needle needs to be repositioned, the cryo-controller 104 may obtain a repositioning indicator from the user. Such indication of repositioning may be obtained in a manner similar to that described above with respect to step 602. If repositioning is needed, the method 600 may move to step 626 during which the monitoring of the needle temperatures and impedances continues and the method then returns to step 604 to re-perform method 600 (or portions thereof). If the needle does not need to be repositioned, the method 600 moves to step 628.

At step 628, the method of repositioning of the needle is complete and the next step of the cryoablation treatment may continue.

The cryoablation apparatuses of the present disclosure may use one or more machine learning models to perform the functions described above. For example, when the cryo-controller adjusts a power signal to the heater and/or supplies a cryogen during a cooling cycle, the cryo-controller may use a cauterization model. The model may use machine learning, artificial intelligence, artificial neural networks or the like to determine an adjustment to made to achieve the cauterization temperature or the movement temperature during a cauterization cycle. The model may be a trained machine learning model that was trained using historical and/or laboratory data to determine a recommended output or power signal based on one or more inputs such as a tissue impedance, needle temperature, current power setting, desired cautery temperature, desired cautery time period, cryogen flow rate, patient information, tissue characteristics, and others.

The term model as used in the present disclosure includes data models created using machine learning and/or artificial intelligence. Machine learning may involve training a mathematical model in a supervised or unsupervised setting. Machine learning models may be trained to learn relationships between various groups of data. The models may be based on a set of algorithms that are designed to model abstractions in data by using a number of processing layers. The processing layers may be made up of non-linear transformations. Machine learning models may include, for example, neural networks, convolutional neural networks and deep neural networks. Such neural networks may be made of up of levels of trainable filters, transformations, projections, hashing, and pooling. The models may be used in large-scale relationship-recognition tasks. The models can be created by using various open-source and proprietary machine learning tools and/or libraries known to those of ordinary skill in the art.

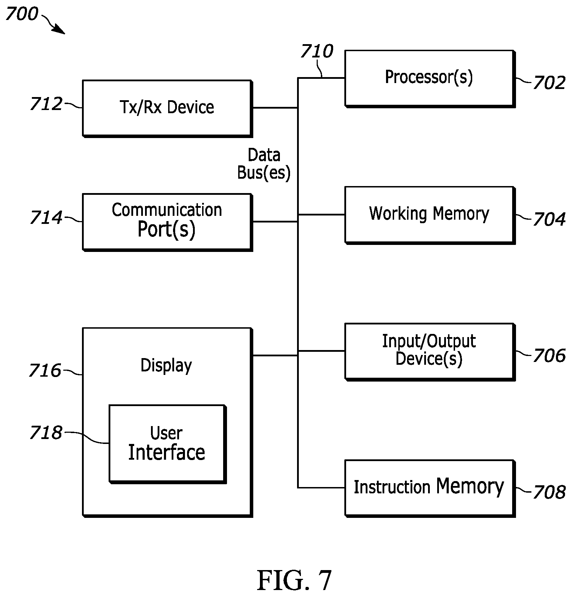

Referring now to FIG. 7, an example computing device 700 is shown. The cryoablation apparatus 100, 200 may include one or more computing devices 700. For example, the cryoablation computing device 102 and/or the cryo-controller 104 may have the elements shown in FIG. 7. The cryoablation control apparatus 214 of the cryoablation apparatus 200 may include one or more computing devices 700. The methods of the present disclosure, such as procedure 500 and/or method 600 may be performed, or steps of such methods may be performed, by a computing device 700.

As shown, the computing device 700 may include one or more processors 702, working memory 704, one or more input/output devices 706, instruction memory 708, a transceiver 712, one or more communication ports 714, and a display 716, all operatively coupled to one or more data buses 710. Data buses 710 allow for communication among the various devices. Data buses 710 can include wired, or wireless, communication channels.

Processors 702 can include one or more distinct processors, each having one or more cores. Each of the distinct processors can have the same or different structure. Processors 702 can include one or more central processing units (CPUs), one or more graphics processing units (GPUs), application specific integrated circuits (ASICs), digital signal processors (DSPs), and the like.

Processors 702 can be configured to perform a certain function or operation by executing code, stored on instruction memory 708, embodying the function or operation. For example, processors 702 can be configured to perform one or more of any function, step, method, or operation disclosed herein.

Instruction memory 708 can store instructions that can be accessed (e.g., read) and executed by processors 702. For example, instruction memory 708 can be a non-transitory, computer-readable storage medium such as a read-only memory (ROM), an electrically erasable programmable read-only memory (EEPROM), flash memory, a removable disk, CD-ROM, any non-volatile memory, or any other suitable memory.

Processors 702 can store data to, and read data from, working memory 704. For example, processors 702 can store a working set of instructions to working memory 704, such as instructions loaded from instruction memory 708. Processors 702 can also use working memory 704 to store dynamic data created during the operation of cryoablation computing device 102. Working memory 704 can be a random access memory (RAM) such as a static random access memory (SRAM) or dynamic random access memory (DRAM), or any other suitable memory.

Input-output devices 706 can include any suitable device that allows for data input or output. For example, input-output devices 706 can include one or more of a keyboard, a touchpad, a mouse, a stylus, a touchscreen, a physical button, a speaker, a microphone, or any other suitable input or output device.

Communication port(s) 714 can include, for example, a serial port such as a universal asynchronous receiver/transmitter (UART) connection, a Universal Serial Bus (USB) connection, or any other suitable communication port or connection. In some examples, communication port(s) 714 allows for the programming of executable instructions in instruction memory 708. In some examples, communication port(s) 714 allow for the transfer (e.g., uploading or downloading) of data, such as cryoprobe operating information, temperature data, impedance data, and the like.

Display 716 can display a user interface 718. User interfaces 718 can enable user interaction with the cryoablation computing device 102. For example, user interface 718 can be a user interface that allows an operator to interact, communicate, control and/or modify different messages, settings, or features that may be presented or otherwise displayed to a user. The user interface 718 can include a slider bar, dialogue box, or other input field that allows the user to control, communicate or modify a setting, limitation or input that is used in a cryoablation treatment. In addition, the user interface 718 can include one or more input fields or controls that allow a user to modify or control optional features or customizable aspects of the cryoablation computing device 102 and/or the operating parameters of the cryoablation system 100. In some examples, a user can interact with user interface 718 by engaging input-output devices 706. In some examples, display 716 can be a touchscreen, where user interface 718 is displayed on the touchscreen. In other examples, display 716 can be a computer display that can be interacted with using a mouse or keyboard. The user interface 300 is an example of the user interface 718.

Transceiver 712 allows for communication with a network. In some examples, transceiver 712 is selected based on the type of communication network cryoablation computing device 102 will be operating in. Processor(s) 702 is operable to receive data from, or send data to, a network, such as wired or wireless network that couples the elements of the cryoablation apparatus 100.

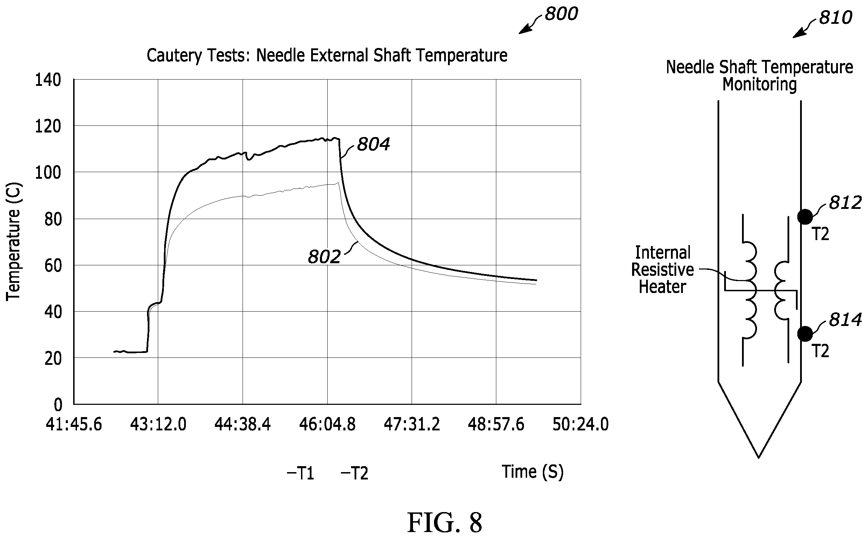

Referring now to FIG. 8, a test was performed using a cryoprobe 810. The cryoprobe 810 included an internal resistive heater such as that described above. A temperature T2 of the cryoprobe 810 at a first location (814) was measured. The first location was located at a position of about 10 mm from a distal end of the tip of the cryoprobe 810. A temperature T1 of the cryoprobe 810 at a second location (812) was also measured. The second location was located at a position of about 20 mm from the distal end of the tip of the cryoprobe 810.

The graph 800 illustrates the temperatures T1 and T2 achieve using the cryoprobe 810. Line 804 illustrates the temperature T2 and line 802 illustrates the temperature T1. At the first location, temperature T2 achieved a temperature in excess of 100 degrees Celsius. At the second location and during approximately the same time period, temperature T1 achieved a temperature in excess of 80 degrees Celsius. These temperatures are sufficient to perform a suitable cauterization cycle. During the cooling of the needle, the cryoprobe 810 was able to drop from the cauterization temperature to a safe movement temperature at or around 50 degrees Celsius.

The following is a list of non-limiting illustrative embodiments disclosed herein:
Illustrative embodiment 1: A method of positioning a cryoprobe comprising: receiving a cryoprobe repositioning selector indicating an desire to reposition a cryoprobe relative to a target tissue; obtaining cryoprobe operating information from one or more sensors positioned in the cryoprobe, the cryoprobe operating information characterizing one or more operating parameters of the cryoprobe; energizing a heater in the cryoprobe to heat a tip of the cryoprobe to a predetermined cautery temperature threshold; and displaying a cautery indicator when the tip of the cryoprobe is greater than or equal to the cautery temperature threshold.
Illustrative embodiment 2. The method of illustrative embodiment 1, further comprising: cooling the tip of the cryoprobe to a temperature less than or equal to a movement temperature; and displaying a movement indicator when the tip of the cryoprobe is less than or equal to the movement temperature.
Illustrative embodiment 3. The method of any of illustrative embodiments 1 or 2, wherein the cautery temperature threshold is about 80 degrees Celsius.
Illustrative embodiment 4. The method of any of illustrative embodiments 1 to 3, wherein the tip of the cryoprobe is maintained at the cautery temperature threshold for a period of about 5 seconds to about 20 seconds.
Illustrative embodiment 5. The method of any of illustrative embodiments 1 to 4, wherein the one or more sensors comprises at least one impedance sensor and at least one temperature sensor, and the cryoprobe operating information comprises impedance data and temperature data.
Illustrative embodiment 6. The method of illustrative embodiment 5, further comprising determining a completion of cauterization of the target tissue at the tip of the cryoprobe based on the impedance data and the temperature data.
Illustrative embodiment 7. The method of any of illustrative embodiments 1 to 6, further comprising displaying a movement indicator indicating when cautery of the target tissue is complete and the cryoprobe can be moved to a new location relative to the target tissue.
Illustrative embodiment 8. The method of any of illustrative embodiments 1 to 7, wherein the step of energizing the heater in the cryoprobe comprises adjusting a power delivered to the heater based on the impedance data and the temperature data.
Illustrative embodiment 9. The method of any of illustrative embodiments 1 to 8, wherein the one or more sensors comprises a plurality of impedance sensors and a plurality of temperature sensors, the plurality of impedance sensors spaced apart from each other to provide impedance data for a plurality of locations along a length of the tip of the cryoprobe, the plurality of temperature sensors spaced apart from each other to provide temperature data for a plurality of locations along a length of the tip of the cryoprobe.
Illustrative embodiment 10. The method of any of illustrative embodiments 1 to 9, further comprising determining a cauterization length based on the impedance data and the temperature data, the cauterization length characterizing a length of the tip of the cryoprobe that reaches the cautery temperature threshold for a cautery period of time.
Illustrative embodiment 11. A cryoablation system comprising: a cryoprobe assembly comprising a needle for insertion at a target tissue; a cryogen delivery apparatus fluidly connected to the cryoprobe assembly; a cryoablation control apparatus comprising a power supply, a user input/output unit, at least one processor, and memory, wherein executable instructions stored in the memory cause the at least one processor to: receive a cryoprobe repositioning selector indicating an desire to reposition the cryoprobe relative to the target tissue; obtain cryoprobe operating information from one or more sensors positioned in the needle, the cryoprobe operating information characterizing one or more operating parameters of the needle; energize a heater in the needle to heat the needle to a predetermined cautery temperature threshold; and display a cautery indicator when a temperature of the needle is greater than or equal to the cautery temperature threshold.
Illustrative embodiment 12. The system of illustrative embodiment 11, wherein the executable instructions further cause the processor to: cool the tip of the cryoprobe to a temperature less than or equal to a movement temperature; and display a movement indicator when the tip of the cryoprobe is less than or equal to the movement temperature.
Illustrative embodiment 13. The system of any of illustrative embodiments 11 or 12, wherein the cautery temperature threshold is about 90 degrees Celsius.
Illustrative embodiment 14. The system of any of illustrative embodiments 11 to 13, wherein the temperature of the needle is maintained at the cautery temperature threshold for a period of about 5 seconds to about 20 seconds.
Illustrative embodiment 15. The system of any of illustrative embodiments 11 to 14, wherein the one or more sensors comprises at least one impedance sensor and at least one temperature sensor, and the cryoprobe operating information comprises impedance data and temperature data.
Illustrative embodiment 16. The system of illustrative embodiment 15, wherein the executable instructions further cause the processor to determine a completion of cauterization of the target tissue based on the impedance data and the temperature data.
Illustrative embodiment 17. The system of any of illustrative embodiments 11 to 16, wherein the executable instructions further cause the processor to display a movement indicator indicating when cautery of the target tissue is complete and the needle can be moved to a new location relative to the target tissue.
Illustrative embodiment 18. The system of any of illustrative embodiments 15 to 17, wherein the step of energizing the heater in the needle comprises adjusting a power delivered to the heater based on the impedance data and the temperature data.
Illustrative embodiment 19. The system of any of illustrative embodiments 11 to 18, wherein the one or more sensors comprises a plurality of impedance sensors and a plurality of temperature sensors, the plurality of impedance sensors spaced apart from each other to provide impedance data for a plurality of locations along a length of the needle, the plurality of temperature sensors spaced apart from each other to provide temperature data for a plurality of locations along a length of the needle.
Illustrative embodiment 20. The system of any of illustrative embodiments 11 to 19, wherein the executable instructions further cause the processor to determine a cauterization length based on the impedance data and the temperature data, the cauterization length characterizing a length of the needle that reaches the cautery temperature threshold for a cautery period of time.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A cryoablation system (100, 200) comprising:
a cryoprobe assembly (112, 202, 810) comprising a needle (114, 206, 400);
a cryogen delivery apparatus (106, 210) fluidly connected to the cryoprobe assembly;
a cryoablation control apparatus (104, 214, ) comprising a power supply, a user input/output unit, at least one processor, and memory, wherein executable instructions stored in the memory cause the at least one processor to:
receive a cryoprobe repositioning selector indicating a desire to reposition the cryoprobe;
obtain cryoprobe operating information from one or more sensors positioned in the needle (114, 400);
energize a heater (204, 410) in the needle to heat the needle to a predetermined cautery temperature threshold; and
display a cautery indicator when a temperature of the needle is greater than or equal to the cautery temperature threshold.

2. The system of claim 1, wherein the executable instructions further cause the processor to: cool the tip (404) of the cryoprobe (112) to a temperature less than or equal to a movement temperature; and display a movement indicator when the tip of the cryoprobe (112, 202) is less than or equal to the movement temperature.

3. The system of claim 1 or 2, wherein the cautery temperature threshold is about 70 degrees Celsius or higher, optionally, the cautery temperature threshold is about 70 to about 100 degrees Celsius.

4. The system of claim 1, 2 or 3, wherein the temperature of the needle (114, 206, 400) is maintained at the cautery temperature threshold for a period of 5 seconds to 20 seconds.

5. The system of any one of the preceding claims, wherein the one or more sensors (412) comprises at least one impedance sensor (412) and at least one temperature sensor, and the cryoprobe operating information comprises impedance data and temperature data.

6. The system of claim 5, wherein the executable instructions further cause the processor to determine a completion of cauterization based on the impedance data and the temperature data.

7. The system of claim 6, wherein the executable instructions further cause the processor to display a movement indicator indicating when cautery of the target tissue is complete and the needle (206, 400) can be moved to a new location.

8. The system of claim 5, 6 or 7, wherein the step of energizing the heater (204, 410) in the needle comprises adjusting a power delivered to the heater based on the impedance data and the temperature data.

9. The system of any one of the preceding claims, wherein the one or more sensors comprises a plurality of impedance sensors (412) and a plurality of temperature sensors, the plurality of impedance sensors spaced apart from each other to provide impedance data for a plurality of locations along a length of the needle, the plurality of temperature sensors spaced apart from each other to provide temperature data for a plurality of locations along a length of the needle.

10. The system of claim 9, wherein the executable instructions further cause the processor to determine a cauterization length based on the impedance data and the temperature data, the cauterization length characterizing a length of the needle that reaches the cautery temperature threshold for a cautery period of time.

11. A method of positioning a cryoprobe (600) comprising:
receiving a cryoprobe repositioning selector (602) indicating a desire to reposition a cryoprobe relative to a target tissue;
obtaining cryoprobe operating information from one or more sensors positioned in the cryoprobe (604), the cryoprobe operating information characterizing one or more operating parameters of the cryoprobe;
energizing a heater (606) in the cryoprobe to heat a tip of the cryoprobe to a predetermined cautery temperature threshold; and
displaying a cautery indicator (616) when the tip of the cryoprobe is greater than or equal to the cautery temperature threshold and, optionally, further comprising: cooling the tip of the cryoprobe (618) to a temperature less than or equal to a movement temperature; and displaying a movement indicator when the tip of the cryoprobe is less than or equal to the movement temperature.

12. The method of claim 11, wherein the cautery temperature threshold is about 80 degrees Celsius or greater and, optionally, the cautery temperature threshold is about 90 to 100 degrees Celsius and, optionally, the tip of the cryoprobe is maintained at the cautery temperature threshold for a period of about 5 seconds to about 20 seconds , optionally, the step of energizing the heater in the cryoprobe comprises adjusting a power delivered to the heater based on the impedance data and the temperature data (612, 614) and, optionally, the method further comprising displaying a movement indicator indicating when cautery of the target tissue is complete and the cryoprobe can be moved to a new location relative to the target tissue

13. The method of claim 11 or 12, wherein the one or more sensors comprises at least one impedance sensor and at least one temperature sensor, and the cryoprobe operating information comprises impedance data and temperature data and, optionally, the method further comprising determining a completion of cauterization of the target tissue at the tip of the cryoprobe based on the impedance data and the temperature data (612).

14. The method of and one of claims 11-13, wherein the one or more sensors comprises a plurality of impedance sensors and a plurality of temperature sensors, the plurality of impedance sensors spaced apart from each other to provide impedance data for a plurality of locations along a length of the tip of the cryoprobe, the plurality of temperature sensors spaced apart from each other to provide temperature data for a plurality of locations along a length of the tip of the cryoprobe and, optionally, further comprising determining a cauterization length based on the impedance data and the temperature data, the cauterization length characterizing a length of the tip of the cryoprobe that reaches the cautery temperature threshold for a cautery period of time.

15. A cryoablation probe (202) including a needle (206, 400 ) and a heater (204, 410) positioned at or near the tip (404) of the needle, the needle including one or more impedance sensor (412) and/or one of more temperature sensor, optionally, the heater extends longitudinally in an axial direction along the length of the needle (114, 206, 400), optionally the heater is from 20-30 mm long.
